# EUROPEAN PATENT SPECIFICATION

(11) **EP 1 434 053 B1**
(45) Date of publication and mention of the grant of the patent: **26.03.2008**
(21) Application number: 02800770.6
(22) Date of filing: 04.10.2002
(51) Int. Cl.: G01N 33/68, G01N 33/92, G01N 33/577, G01N 33/53

(54) **REAGENT FOR DETECTING RISK FACTOR FOR ALZHEIMER'S DISEASE, DETECTION KIT THEREFOR AND METHOD OF DETECTING RISK FACTOR FOR ALZHEIMER'S DISEASE USING THE SAME**
REAGENS ZUM NACHWEIS EINES MORBUS-ALZHEIMER-RISIKOFAKTORS, NACHWEISKIT DAFÜR UND VERFAHREN ZUM NACHWEIS EINES MORBUS-ALZHEIMER-RISIKOFAKTORS UNTER VERWENDUNG DAVON
REACTIF POUR DETECTER UN FACTEUR DE RISQUE DE LA MALADIE D'ALZHEIMER, NECESSAIRE DE DETECTION A CET EFFET, ET PROCEDE DE DETECTION DU FACTEUR DE RISQUE DE LA MALADIE D'ALZHEIMER AU MOYEN DE CE NECESSAIRE

(30) Priority: 04.10.2001 JP 2001308465
(43) Date of publication of application: 30.06.2004
(73) Proprietor: Immuno-Biological Laboratories Co., Ltd., Takasaki-shi, Gunma 370-0831 (JP)
(72) Inventor: OTA, Shigeo, Machida-shi, Tokyo 194-0032 (JP); MAEDA, Masahiro, Gunma 370-0831 (JP)
(74) Representative: Hartz, Nikolai
(86) International application number: PCT/JP2002/010363
(87) International publication number: WO 2003/031971

(56) References cited:
- JP-A- 10 197 528
- US-A- 5 716 828
- US-A- 5 773 220
- US-A1- 2001 019 821
- PATENT ABSTRACTS OF JAPAN vol. 1998, no. 12, 31 October 1998 (1998-10-31) & JP 10 197528 A (FUJIREBIO INC), 31 July 1998 (1998-07-31)

## Description

### TECHNICAL FIELD

The present invention relates to a reagent for detecting a risk factor of Alzheimer's disease, a detection kit, and a method for detecting a risk factor of Alzheimer's disease using the reagent and kit. More particularly, the present invention relates to a reagent for detecting a risk factor of Alzheimer's disease using an antibody capable of accurately and easily detecting an apolipoprotein E4 genotype which is a risk factor of Alzheimer' s disease, a detection kit, and a detection method using the reagent and kit.

### BACKGROUND ART

Alzheimer's disease was first reported in 1907 by A. Alzheimer, a German neuropathologist. The disease has now become one of the important causes for senile dementia. The percentage of dementia patients among elderly persons above 65 years old in Japan is about 7% at the present time and the number of patients is increasing every year. Not only is the disease a cause for the increase in the medical expense for the elderly, but also a cause for the increase in the national economic burden for caring for the patients.

In view of this situation, general practitioners are also required to positively diagnose and treat dementia patients at an early stage.

Alzheimer's disease rapidly destroys the patients after the onset of symptoms. The disease causes a multiple and conspicuous functional disorder in the high brain cortical layer. The patients exhibit symptoms of aphasia, agnosia, and apraxia at a comparatively early stage. Pathologically, the brain shrinks remarkably and the weight decreases to 900 g or less in the last stage. Histologically, characteristic pathological changes such as disappearance of nerve cells, neurofibrillary tangle, and senile plaques are observed.

Four genes causing hereditary Alzheimer' s diseases shown in the following Table 1 have been so far reported. The proteins are biosynthesized from the genes. These proteins are referred to as "proteins encoded by genes,"

**Table 1**

| Gene | Age of onset | Chromosome seat | Proteins encoded by genes |
|---|---|---|---|
| AD1 | 40-45 | 21^{st} chromosome | Amyloid precurpor protein |
| AD2 | 65 or more | 19^{th} chromosome | Apolipoprotein E |
| AD3 | 30-40 | 14^{th} chromosome | Presenilin-1 |
| AD4 | 55-65 | 1^{st} chromosome | Presenilin-2 |

Among these proteins, apolipoprotein E gene (AD2) is a gene encoding an apolipoprotein (sometimes referred to as "Apo" hereinafter) E which is one of the components of lipoproteins in human blood (a complex of lipid and protein). This gene is presumed to be an Alzheimer' s dementia sensible gene or a risk factor of Alzheimer's disease.

The ApoE consists of 299 amino acids. An ApoE type having cysteine for the amino acids at 112 and 158 from the N-terminal is ApoE2, an ApoE type having cysteine at 112 and arginine at 158 from the N-terminal is ApoE3, and an ApoE type having arginine for both the amino acids at 112 and 158 from the N-terminal is ApoE4. The genotypes of the gene encoding these ApoEs are present as one of the homozygotes or heterozygotes, with a combination of (ApoE2/ApoE2), (ApoE2/ApoE3), (ApoE2/ApoE4), (ApoE3/ApoE3), (ApoE3/ApoE4), or (ApoE4/ApoE4). All human genotypes belong to any one of these homozygotes and heterozygotes.

Investigation on the genotype of sporadic Alzheimer patients due to aging revealed that the Alzheimer's disease occurs most frequently among persons with a genotype of (ApoE4/ApoE4) or (ApoE3/ApoE4). As opposed to these genotypes, the number of patients is small in the genotypes including ApoE2, (ApoE2/ApoE2), (ApoE2/ApoE3), and (ApoE2/ApoE4). The antioxidation effect is in the order of ApoE2>ApoE3>ApoE4. ApoE4 is presumed to promote fibrosis of amyloid β-proteins.

Among the dimer structures forming ApoE, those containing one ApoE4, that is, heterozygotes (ApoE2/ApoE4) and (ApoE3/ApoE4) are reported to induce Alzheimer's disease almost 10 years earlier than the other genotypes, whereas the homozygote (ApoE4/ApoE4) is reported to hasten the disease onset by about 20 years. Although the ApoE4 gene frequency among Japanese is about 10%, the frequency is about 30% among persons suffering from Alzheimer's disease.

Thus, if ApoE4 is expressed on the AD gene, Alzheimer's disease is easily induced, specifically, the ApoE4 is a risk factor of Alzheimer's disease. The method for retarding the onset of Alzheimer's disease by early discovery of this risk factor and for easing symptoms of the disease by dosing a cognitive function improver for Alzheimer type senile dementia have been demanded.

As the method for inspecting these genotypes, the gene amplification inspection represented by the PCR (polymerase chain reaction) method is presently used. This method is a most reliable method in respect of the specificity of identifying a genotype. However, this method requires cells (leukocytes etc.) possessing the gene, a special gene amplification apparatus for detecting the gene at a sufficient sensitivity, and a long time (e.g. half a day) and high cost for the assay. A simple and inexpensive assay method that can replace this method has been desired.

An object of the present invention is, therefore, to provide an assay method which can detect ApoE4 genotypes inexpensively at a sufficient sensitivity as compared with the PCR method presently used for gene inspection and a method for detecting a risk factor of Alzheimer's disease.

US 5,716,828 discloses a method and kit for diagnosing or prognosing Alzheimer's disease, comprising detecting apolipoprotein E4 using an antibody. JP 10-197528 discloses antibodies for determining an apolipoprotein E phenotype. US 2001/0019821 discloses a method for analyzing a sample for the concentration of HIV antibody.

### DISCLOSURE OF THE INVENTION

The present inventor investigated a method for detecting only ApoE4 from ApoE2, ApoE3 or ApoE4 that are structurally very similar each other. As a result, the inventor has found that an antibody against a specific peptide specifically reacts with ApoE4 and that the presence of a risk factor of Alzheimer's disease can be identified by using this antibody.

The inventor has further found that if an antibody against all of ApoE2, ApoE3 and ApoE4 is combined with the above specific antibody against ApoE4, the ApoE4 can be detected at a higher accuracy, a large number of samples can be assayed at a very low cost per one sample as compared with the cost for genetic screening, and a conventional automatic colorimetric apparatus widely used in inspection rooms in hospitals can be used without requiring any special apparatus. These findings have led to the completion of the present invention.

The present invention further provides a kit for detecting a risk factor of Alzheimer's disease containing the following reagents (a) and (b):
(a) a reagent containing an antibody specific to apolipoprotein E4 that does not recognize apolipoprotein E2 and apolipoprotein E3 and
(b) a reagent containing an antibody specific to all of apolipoprotein E2, apolipoprotein E3, and apolipoprotein E4, which recognizes a polypeptide having the peptide sequence of Sequence ID No.3.

The present invention further provides a method for detecting a risk factor of Alzheimer's disease utilizing the above-described reagent or kit.

### BRIEF DESCRIPTION OF THE DRAWINGS

Figure 1 shows specificity of the antibodies prepared in Example 1 and Example 2.
Figure 2 shows the results of identification of genotypes obtained by the absorbance method and the PCR method in Example 4.

### BEST MODE FOR CARRYING OUT THE PRESENT INVENTION

The antibody specific to an apolipoprotein E4 (ApoE4) used in the present invention is an antibody that can recognize only ApoE4, but does not recognize ApoE2 and ApoE3 having a peptide structure similar to the ApoE4. Such an antibody can be prepared by synthesizing an oligopeptide with an adequate length having a sequence specific to only to ApoE4 as a major part by comparing the proteins encoded by the genes of ApoE2 and ApoE3 with the protein encoded by the gene of ApoE4 (the amino acid sequences of ApoE2, ApoE3, and ApoE4 are registered in the NCBI database as accession Nos. 230118, AAH03557, and AAB59397, respectively) and preparing an antibody for the oligopeptide according to a conventional method.

As a specific example of the oligopeptide with an adequate length having a sequence specific only to ApoE4 as a major part, a synthetic peptide having an amino acid sequence of CADMEDVRGRLV (Sequence ID No. 2) prepared by bonding cysteine (C) to the N-terminal side of ADMEDVRGRLV (Sequence ID No. 1), which is an amino acid sequence from No. 106 to No. 116 of ApoE4, can be given. This synthetic peptide (hereinafter referred to as ApoE4 fragment peptide) can be used for preparing the antibody.

The ApoE4 fragment peptide can be obtained by various methods without any specific limitations. For example, the peptide can be prepared by a method known in the art.

This ApoE4 fragment peptide is then combined to a biological polymer compound, which is used as an antigen to produce an antibody specific to ApoE4.

Here, as examples of the biological polymer compound, keyhole limpet haemocyanin (hereinafter referred to as "KLH"), AT ovalbumin (hereinafter referred to as "OVA"), bovine serum albumin (hereinafter referred to as "BSA"), rabbit serum albumin (hereinafter referred to as "RSA"), and thyroglobulin can be given. Of these, KLH and thyroglobulin are preferable.

The ApoE4 fragment peptide can be bonded to a biological polymer compound by a known method such as a mixed acid anhydride method (B. F. Er. Langer et al., J. Biol. Chem. 234, 1090-1094 (1954)) or an activation ester method (A. E. KARU et al., J. Agric. Food Chem. 42, 301-309 (1994)).

A mixed acid anhydride used in the mixed acid anhydride method can be obtained by subj ecting the ApoE4 fragment peptide to the conventional Schotten-Baumann reaction. The resulting mixed acid anhydride is reacted with a biological polymer compound to produce the target combined product of the peptide and the biological polymer compound. As examples of haloformic acid esters used in the mixed acid anhydride method, methyl chloroformate, methyl bromoformate, ethyl chloroformate, ethyl bromoformate, isobutyl chloroformate can be given. The ratio of the peptide, haloformic acid ester, and polymer compound used in this method can be appropriately determined from a wide range.

The Schotten-Baumann reaction is carried out in the presence of a basic compound. Any basic compound commonly used in the Schotten-Baumann reaction can be used in the reaction of the present invention. For example, organic bases such as triethylamine, trimethylamine, pyridine, dimethylaniline, N-methylmorpholine, DBN, DBU and DABCO; and inorganic bases such as potassium carbonate, sodium carbonate, potassium hydrogencarbonate and sodium hydrogencarbonate can be used.

The reaction is usually carried out at a temperature from -20°C to 100°C, preferably from 0°C to 50°C, for 5 minutes to 10 hours, and preferably for 5 minutes to 2 hours.

The reaction of the resulting mixed acid anhydride with a biological polymer compound is usually carried out at a temperature from -20°C to 150°C, preferably from 0°C to 100°C, for 5 minutes to 10 hours, and preferably for 5 minutes to 5 hours. The mixed acid anhydride method is generally carried out in a solvent. Any solvent commonly used for the mixed acid anhydride method can be used. Specific examples are halogenated hydrocarbons such as dichloromethane, chloroform and dichloroethane; aromatic hydrocarbons such as benzene, toluene and xylene; ethers such as diethyl ether, dioxane, tetrahydrofuran and dimethoxyethane; esters such as methyl acetate and ethyl acetate; non-polar solvents such as N,N-dimethylformamide, dimethylsulfoxide and hexamethylphosphoric acid triamide.

On the other hand, the activated ester method can be generally carried out as follows. First, the synthetic peptide is dissolved in an organic solvent and reacted with N-hydroxysuccinic acid imide in the presence of a coupling agent to produce N-hydroxysuccinic acid imide activated ester.

As the coupling agent, any coupling agents commonly used in a condensation reaction such as dicyclohexylcarbodiimide, carbonyldiimidazole, and water-soluble carbodiimide can be used. As the organic solvent, N,N-dimethylformamide (DMF), dimethylsulfoxide, dioxane, and the like can be used. The molar ratio of the peptide and N-hydroxysuccinic acid imide is preferably 1:10-10:1, and ideally 1: 1. The reaction is carried out at a temperature of from 0°C to 50°C, preferably from 22°C to 27°C, for 5 minutes to 24 hours, and preferably for 1 to 2 hours. The reaction temperature for each reaction may be in the range from the melting point to the boiling point of the components to be reacted.

After the coupling reaction, the reaction mixture is added to a solution of the biological polymer compound. If the biological polymer compound has a free amino group, for example, an acid-amide bond is formed between the amino group and the carboxyl group of the peptide. The reaction is carried out at a temperature from 0°C to 60°C, preferably from 5°C to 40°C, and more preferably from 22°C to 27°C, for 5 minutes to 24 hours, preferably for 1-16 hours, and more preferably for 1-2 hours.

The reaction product obtained by any one of the above methods is purified by dialysis or using a desalting column or the like to produce a combination of the ApoE4 fragment peptide and the polymer compound (hereinafter referred to simply as "combined product").

Next, a method for preparing an antibody using the combined product obtained in this manner as an antigen and the immunochemical assay method using this antibody will be described. In preparing the antibody, known methods such as a method described in publications, for example, Lecture of Biochemistry Experiment, Immunobiochemical Research Method (edited by The Japanese Biochemical Society) can be appropriately applied.

To prepare the antibody specific to ApoE4 of the present invention using the above combined product, an animal is immunized with the combined product and the antibody is extracted from the animal.

Specifically, for example, the combined product such as a combination of ApoE4 fragment peptide and thyroglobulin is first dissolved in a sodium phosphate buffer solution (hereinafter referred to as "PBS"). The solution is combined with an adjuvant such as a Freund's complete adjuvant, an incomplete adjuvant, or alum and the resulting mixture is used as an immune source to immunize a mammal.

Any animal commonly used in the field concerned, for example, a mouse, rat, rabbit, goat, or horse, can be used as the immunized animal. Any method such as hypodermic injection, interperitoneal injection, intravenous injection, hypodermic injection, or muscle injection can be used for immunizing the animal, with hypodermic injection and interperitoneal injection being preferable. Immunization may be carried out one of more times, preferably several times at intervals of 1-5 weeks.

Then, blood is extracted from the immunized animal using a conventional method. The antibody against the ApoE4 fragment peptide can be obtained using the serum separated from the blood.

Alternatively, immunized cells obtained by immunizing the animal using the combined product may be fused with myeloma cells to obtain a hybridoma. The antibody against the ApoE4 fragment peptide can also be extracted from a culture product of the hybridoma.

The antibody obtained in this manner may be labeled as required. Enzymes such as horseradish peroxidase (hereinafter referred to as "HRP") and alkaline phophataze, fluorescent substances such as fluoresceine isocyanate and Rhodamine, radioactive materials such as ³²P and ¹²⁵I, chemoluminescence substances and the like can be used as a labeling substance.

The ApoE4 fragment peptide labeled with these labeling substances may be used in the immunochemical assay method.

The reagent for detecting a risk factor of Alzheimer's disease of the present invention (hereinafter referred to as "invention reagent") can be prepared by combining the above-mentioned antibody and a carrier. As examples of the carrier, a glass tube, polystyrene beads, a microtiter plate, and a membrane can be given. The invention reagent may be either a liquid or a freeze dried product which can be converted into a liquid by the addition of water when used.

On the other hand, as examples of the sample which may contain a risk factor of Alzheimer's disease to be detected using the invention reagent, various body fluids such as whole blood, blood serum and plasma can be given. The sample may be subjected to the assay after appropriately diluting, if necessary. A buffer solution containing phosphoric acid, carbonic acid, or Tris and a physiological saline solution can be used for the dilution. As specific examples of the sample dilution, in the case of whole blood, a 51-fold dilution (500 µl of a buffer solution for dilution is added to 10 µl of the whole blood) and in the case of blood serum or plasma, 101-fold dilution (1000 µl of a buffer solution for dilution is added to 10 µl of blood serum or plasma) can be given.

The method for detecting a risk factor of Alzheimer's disease of the present invention (hereinafter referred to as "invention method") comprises adding the invention reagent to a sample and detecting the antigen-antibody reaction of the antibody in the invention reagent. As the method for detecting the antigen-antibody reaction of the invention method, a commonly used method such as an RIA method (radioactive isotope immunity assay method), ELISA method (Engvall, E, Methods in Enzymol, 70, 419-4439 (1980)), immunity nephelometry, fluorescent antibody technique, plaque method, sport method, condensation method, and ouchterlony method can be used (Hybridoma and Monoclonal Antibody, R&D Planning Co., 30-53, March 5, 1982).

As a method for carrying out the invention method more advantageously, a sandwich method in which the ApoE4 to be detected is sandwiched between two types of antibodies can be mentioned. As one of the antibodies used in this method, the above-described antibody specific to ApoE4 can be used, with the other antibody being an antibody against all of the ApoE2, ApoE3 and ApoE4.

There are no specific limitations to the antibody against all of the ApoE2, ApoE3 and ApoE4 used in the present invention inasmuch as such an antibody recognizes a polypeptide having a peptide sequence of Sequence ID No.3 and can specifically recognized all of the ApoE2, ApoE3 and ApoE4. Such an antibody can be prepared by synthesizing a protein corresponding to a sequence common to all of the gene sequences of ApoE2, ApoE3 and ApoE4 and preparing an antibody for the peptide. For example, a synthetic peptide having an amino acid sequence of CEKVQAAVGTSAAPVPSDNH (Sequence ID No. 4) is prepared by bonding cysteine (C) to the N-terminal side of EKVQAAVGTSAAPVPSDNH (Sequence ID No. 3) which is a sequence of amino acids from No. 281 to No. 299 of the amino acid sequence common to ApoE2, ApoE3 and ApoE4.

The procedure for detecting ApoE4 will now be explained taking the case of the sandwich method. (a) First, the antibody against all of the ApoE2, ApoE3 and ApoE4 is immobilized on a carrier. (b) Then, the surface of the carrier on which the antibody is not immobilized is blocked with a substance irrelevant to the antigen, for example, BSA. (c) After producing an ApoE-antibody complex by adding a diluted sample containing ApoE, (d) an antibody specific to the ApoE4 bonded to a labeled enzyme is added to react with the ApoE-immobilized antibody complex. (e) Finally, the amount of color generation or the like of the labeled enzyme is measured to determine the amount of ApoE4 in a sample using a previously prepared calibration curve.

In the step (a), any carriers commonly used in the ELISA method can be used as the carrier on which the antibody is immobilized without any specific limitations. A 96-well microtiter plate made from polystyrene or an amino group bonding type microtiter plate can be given as examples. To immobilize an antibody, a buffer solution containing the antibody is added to the carrier and incubated. As the buffer solution, a known buffer solution such as a 10 mM PBS can be used. The concentration of the antibody in the buffer solution can be selected from a wide range usually from 0.01 to 100 µg/ml, and preferably from 0.1 to 20 µg/ml. When the 96-well microtiter plate is used, the amount of the antibody solution is less than 300 µl/well, and preferably 20-150 µl/well. Although there are no specific limitations to the incubating conditions, usually incubation overnight at about 4°C is preferable.

Blocking in the step (b) is carried out to prevent ApoE in a later-added sample from being adsorbed in the carrier on which the antibody has been immobilized irrespective of an antigen-antibody reaction. As the blocking agent, other than BSA and a skim milk solution, commercially available products such as block ace (Code No. UK-25B, manufactured by Dainippon Pharmaceutical Co., Ltd.), for example, can be used. As a specific blocking method, a method of adding an appropriate amount of 1% BSA solution to the immobilized part of the antigen, incubating overnight at 4°C, and washing with a buffer solution can be given. As a buffer solution used in this method, a 50 mM PBS (pH 7.2) containing 0.05% (v/v) Tween-20 and 0.05% NaN₃ is preferable.

Subsequently, in the step (c), the sample containing ApoE is caused to come into contacted with the immobilized antibody to supplement ApoE with the immobilized antibody, thereby producing an ApoE-immobilized antibody complex. Although there are no specific limitations, the reaction can be carried out for about one hour at about 37°C. After the reaction, it is preferable to wash the carrier with a buffer solution to remove unreacted proteins and the like. A 10 mM PBS (pH 7.2) containing 0.05% (v/v) Tween-20 is preferable as a buffer solution used in this reaction.

Furthermore, in step (d), an antibody specific to the ApoE4 labeled with a labeling substance is added to the ApoE supplemented with the immobilized antibody to form a complex of the immobilized antibody, ApoE, and the antibody specific to the ApoE4 labeled with a labeling substance. After the reaction, it is preferable to wash the carrier with a buffer solution to remove unreacted proteins and the like. The above-mentioned buffer solutions can be used as the buffer solution in this reaction. The antibody specific to ApoE4 labeled with a labeling substance is preferably reacted with the immobilized antibody bonded with the carrier after diluting to about a 5, 000-fold to 10, 000-fold, preferably to the ultimate absorbance of 1.0-1.5. A buffer solution can be used for dilution. The antibody specific to ApoE4 labeled with a labeling substance bonds only to ApoE4 among ApoEs supplemented with the immobilized antibody by the above reaction. ApoE4 can be detectable by detecting the labeling substance in this complex.

As examples of the labeling substance used in the above step (d), enzymes such as horseradish peroxidase and alkaline phophataze, fluorescent substances such as fluoresceine isocyanate and Rhodamine, radioactive materials such as ³²P and ¹²⁵I, and chemoluminescent substances can be given. For example, when a peroxidase is used as an enzyme, a coloring substrate solution containing hydrogen peroxide and 3,3',5,5'-tetramethyl benzidine (hereinafter referred to as "TMB") or o-phenylenediamine (hereinafter referred to as "OPD") can be used. Although there are no specific limitations, the coloring reaction is carried out at about 25°C for about 20 minutes after adding the coloring substrate solution. The reaction is terminated with the addition of 2N sulfuric acid. When OPD is used, the absorbance at 492 nm is measured, and when TMB is used, the absorbance at 450 nm is measured. On the other hand, when alkaline phosphatase is used as the enzyme to bond to the second antibody, a method of using p-nitrophenyl phosphate as a coloring substrate, terminating the enzyme reaction with the addition of 2N NaOH, and measuring the absorbance at 415 nm is suitable.

The concentration of ApoE4 in a sample can be calculated using a calibration curve previously prepared using the absorbances of reaction solutions with a known ApoE4 concentration.

Moreover, according to the present invention, the genotype of ApoE in a sample can be identified by using a standard solution 1 which can identify an ApoE4 homogeneous genotype and an ApoE4 heterogeneous genotype (hereinafter referred to simply as "standard solution 1") and a standard solution 2 which can identify an ApoE4 heterogeneous genotype and genotypes other than ApoE4 (hereinafter referred to simply as "standard solution 2") in the above-mentioned detection method.

The concentration of the standard solution 1 is determined from the concentration of ApoE4 contained in samples such as various body fluids, whole blood, blood serum, and plasma between the minimum concentration of ApoE4 of a subject having an ApoE4/E4 homogenous genotype of which all ApoEs contained in a sample are ApoE4 and the maximum concentration of ApoE4 of a subject having ApoE4 heterogeneous genotype of which the ApoEs contained in a sample are ApoE4 and ApoE3, or ApoE4 and ApoE2.

The concentration of the standard solution 2 is also determined from the concentration of ApoE4 contained in samples between the minimum concentration of ApoE4 of a subject having ApoE4 heterogeneous genotype of which the ApoEs contained in a sample are ApoE4 and ApoE3, or ApoE2 and ApoE4 and the maximum concentration of ApoE4 of a subject having a genotype other than ApoE4 of which all ApoEs contained in a sample are ApoE3, all ApoEs are ApoE2, or all ApoEs are ApoE3 and ApoE2.

Specifically, when whole blood is used as a sample, the standard solution 1 and standard solution 2 are prepared from solutions with an ApoE4 concentration respectively of 1.2 µg/ml or 0.24 µg/ml by diluting these solutions to the same dilution magnification as applied to the whole blood when measurement is performed.

More specifically, when A is an absorbance of the standard solution 1, B is an absorbance of the standard solution 2, and C is an absorbance of ApoE4 in a sample, the genotype of ApoE can be identified as follows.
When A < C, the sample has an ApoE4/E4 homogeneous genotype. When B < C < A, the sample has an ApoE4 heterogeneous genotype (ApoE4/E3 or ApoE4/E2 genotype).
When C < B, the sample has a genotype other than the ApoE4 genotype (ApoE3/E3, ApoE3/E2, or ApoE2/E2 genotype).
As a matter of course, these measurements may be accompanied by a reagent blank test and correction of absorbance.

In addition, to easily carry out the method of the present invention, a kit for detecting a risk factor of Alzheimer's disease can be used. The detection kit consists of the following combinations, for example.

A combination of the following reagents (a) and (b):
(a) a reagent containing an antibody against ApoE4 that does not recognize apolipoprotein E2 and E3 and
(b) a reagent containing an antibody against all of ApoE2, ApogE3, and ApoE4, which recognizes a polypeptide having the peptide sequence of Sequence ID. No. 3.

A combination of the following reagents (c) and (d):
(c) a reagent containing a labeled antibody against ApoE4 that does not recognize apolipoprotein E2 and E3 and
(d) a reagent containing a immobilized antibody against all of ApoE2, ApogE3, and ApoE4, which recognizes a polypeptide having the peptide sequence of Sequence ID. No. 3.
A combination of the above detection kit and the following standard solutions:
(e) a standard solution 1 which can identify an ApoE4 homogeneous genotype and an ApoE4 heterogeneous genotype and
(f) a standard solution 2 which can identify an ApoE4 heterogeneous genotype and genotypes other than ApoE4.

ApoE4 in a sample can be detected correctly and simply by using the detection reagent, method, and kit of the present invention. The presence of ApoE4 and its amount in a sample indicate that the possession of the ApoE4 gene by the living body suggests a high possibility of the living body contracting Alzheimer's disease, i.e., the possession of a risk factor of Alzheimer's disease.

### EXAMPLES

The present invention will now be described in detail by way of examples, which should not be construed as limiting the present invention.

### Example 1

### Preparation of an antibody specifically recognizing ApoE4

The antibody specifically recognizing ApoE4 was prepared using a synthetic peptide (hereinafter referred to as "ApoE4 fragment peptide") having an amino acid sequence of CADMEDVRGRLV (Sequence ID No. 2) having cysteine (C) bonded to the amino acid sequence from No. 106 to No. 116 of the N-terminal side of ApoE4.

### (1) Preparation of an antigen for immunity

The combined product of the ApoE4 fragment peptide (manufactured by Synpep) and thyroglobulin used as immunogen was prepared by the EMCS (N-(6-maleimidocaproyloxy)-succinimide) method. In preparing the combined product, thyroglobulin, ApoE4 fragment peptide, and EMCS were used at a molar ratio of 1:300:400. 5 mg of thyroglobulin was dissolved in 1 ml of a 0.01 M phosphate buffer solution. 80 g/µl EMCS dissolved in dimethylformamide in the amount satisfying the above molar ratio was added to obtain a thyroglobulin-EMCS complex. A solution of ApoE4 fragment peptide prepared by dissolving 4 mg of the ApoE4 fragment peptide in about 1 ml of distilled water was added to the solution containing this complex in the amount satisfying the above molar ratio to obtain a complex of the ApoE4 fragment peptide and thyroglobulin cross-linked by EMCS. The resulting mixture was dialyzed using PBS to make a concentration of 1.0 g/µl, thereby obtaining an antigen for immunity.

### (2) Immunity sensitization

Rabbits and mice were immunized using the antigen for immunization obtained in (1) above. Rabbits were immunized using 100 µl (100 µg) at an interval of one week or two weeks. Mice were immunized using 50 µl (50 µg) at an interval of one week. The antigens for both the rabbits and mice were mixed with a Freund's complete adjuvant only at the time of first immunization and with a Freund's incomplete adjuvant from the second immunization and thereafter. Blood was collected from the rabbits after immunization eight times and from the mice after immunization four times.

### (3) Preparation of an antigen for screening

A solution of ApoE4 fragment peptide (manufactured by Synpep) in distilled water was used as an ApoE4 fragment peptide antigen for screening. A solution of ApoE2, ApoE3 and ApoE4 (manufactured by Wako Pure Chemical Industries, Ltd.) in distilled water was used as an ApoE antigen for screening. In addition, ApoE4 (manufactured by Wako Pure Chemical Industries, Ltd.) was used as a standard solution also in Example 3.

### (4) Reactivity of antiserum with the ApoE4 fragment peptide

The reactivity of the antiserum prepared in (2) above was investigated by the ELISA method using the ApoE4 fragment peptide antigen for screening prepared in (3) above. The ApoE4 fragment peptide for screening prepared in (3) above was diluted with a 0.1 M carbonic acid buffer (pH 9.5) to a concentration of 1.0 µg/ml and implanted in a 96-well plate in the amount of 50 µl per well. The plate was washed with PBS, blocked with a 0.1% BSA/PBS/0.05% NaN₃ solution. A 100-fold diluted solution of the antiserum was diluted to a volume of two-fold in series, each diluted solution was added to the wells, in an amount of 50 µl/well, and reacted at 37°C for 30 minutes. After the reaction, the plate was washed four times with 0.05% Tween 20-PBS. An HRP labeled anti-rabbit IgG (manufactured by IBL) was added to the rabbit antiserum and an HRP labeled anti-mouse IgG (manufactured by IBL) was added to the mouse antiserum, each in the amount of 50 µl/well, and reacted at 37°C for 30 minutes. After the reaction, a solution prepared by dissolving ortho phenylenediamine (OPD) in a 0.05 M phosphate-citrate buffer solution (pH 4.5) containing a 0.03% hydrogen peroxide solution to a concentration of 0.4 mg/ml was added in an amount of 100 ul/well. The mixture was allowed to stand for 15 minutes at room temperature under shaded conditions to cause the mixture to produce a color. After color production, 100 µl of 1N sulfuric acid was added to each well to terminate the reaction. The absorbance at 490 nm was measured to confirm the reactivity of the antiserums with the ApoE4 fragment peptide.

### (5) Reactivity of an antiserum with ApoE4

The reactivity of the antiserum was investigated by the Western blotting method using the ApoE antigen for screening prepared in (3) above. First, the antigen was applied to electrophoresis on a 12% acrylamide gel at 20 mA. The gel was blotted onto a membrane, followed by blocking with 3% skimmed milk/1% BSA/PBS/0.05% NaN₃ at 37°C for two hours. After washing with 0.05% Tween 20-PBS, each antiserum was diluted with 0.1% Tween 20-PBS to 200-fold and reacted overnight at 4°C. After the reaction, the plate was washed with 0.05% Tween 20-PBS. An HRP labeled anti-rabbit IgG was added to the rabbit antiserum and an HRP labeled anti-mouse IgG was added to the mouse antiserum, followed by the reaction at 37°C for 1 hour. After the reaction, the reaction product was washed with 0.05% Tween 20-PBS. A color was produced using ECL (manufactured by Amersham Pharmacia Biotech) to sensitize an X-ray film. The reactivity of the antiserums and ApoE4 was confirmed in this manner.

### (6) Preparation of hybridoma cells

A monoclonal antibody to the mouse of which the antiserum potency and the reactivity with ApoE4 have been confirmed in (5) above was prepared. First, spleen cells and mouse myeloma cells (X63-Ag8) of a mouse of which the activity of the anti-ApoE4 fragment peptide antibody in the blood serum had been increased were fused by cell fusion using the PEG method. The antibody activity against the ApoE4 fragment peptide in a culture broth supernatant liquid in which cell growth has been confirmed was investigated using the following method. The reactivity of the culture broth supernatant liquid was investigated by the ELISA method using the ApoE antigen for screening prepared in (3) above. First, the ApoE antigen for screening prepared in (3) above was diluted with a 0.1 M carbonic acid buffer (pH 9.5) to a concentration of 1.0 µg/ml and implanted in a 96-well plate in an amount of 50 µl per well. The plate was washed with PBS, blocked with a 0.1% BSA/PBS/0.05% NaN₃ solution. The culture broth was added to the plate in an amount of 50 µl/well and reacted at 4°C overnight. After the reaction, the plate was washed four times with 0.05% Tween 20-PBS. An HRP labeled anti-mouse IgG was added in an amount of 50 µl/well and the mixture was reacted at 37°C for 30 minutes. After the reaction, a solution prepared by dissolving OPD in a 0.05 M phosphate-citrate buffer solution (pH 4.5) containing a 0.03% hydrogen peroxide solution to a concentration of 0.4 mg/ml was added in an amount of 100 µl/well. The mixture was allowed to stand for 15 minutes at room temperature under shaded conditions to cause the mixture to produce a color. After color production, 100 µl of 1N sulfuric acid was added to each well to terminate the reaction. The absorbance at 490 nm was measured. Wells in which a specific antibody activity was identified were selected. Cells in the selected wells were cloned using the limiting dilution method. As a result, a hybridoma cell strain producing an anti-ApoE4 fragment peptide antibody specifically reacting with ApoE4 was cloned.

### (7) Preparation of combined product of an anti-ApoE4 fragment peptide antibody and HRP

A combined product of an anti-ApoE4 fragment peptide antibody and HRP was prepared as follows. 20 mg of the anti-ApoE4 fragment peptide antibody prepared above was digested with pepsin and filtered by gel filtration to purify F (ab')₂ fragments of an anti-ApoE4 fragment peptide antibody. The F(ab')₂ fragments were reduced to Fab' fragments using 2-mercaptoethanol. HRP and EMCS were reacted for 60 minutes at 37°C and the reaction product was filtered by gel filtration to obtain an HRP-EMCS combined product. This combined product was reacted with the Fab' fragments at 4°C overnight. The reaction product was filtered by gel filtration and cross-linked with EMCS to obtain a combined product of the anti-ApoE4 fragment peptide antibody and HRP.

### Example 2

### Preparation of an antibody against all of ApoE2, ApogE3, and ApoE4

The antibody against all of ApoE2, ApogE3 and ApoE4 was prepared in the same manner as in Example 1 using a synthetic peptide having an amino acid sequence of CEKVQAAVGTSAAPVPSDNH (Sequence ID No. 4) having cysteine (C) bonded to the N-terminal side of a sequence of amino acids from No. 281 to No. 299 of the amino acid sequence common to ApoE2, ApoE3 and ApoE4.

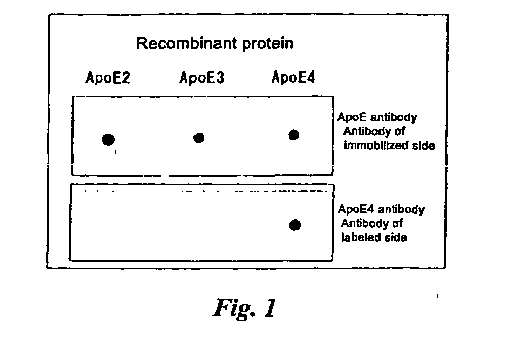

The specificities of the antibodies prepared in Example 1 and Example 2 are shown in Figure 1. As is clear from Figure 1, the antibody prepared in Example 1 is reactive only with ApoE4, whereas the antibody prepared in Example 2 was reactive with all of ApoE2, ApoE3 and ApoE4.

### Example 3

### Construction of the sandwich ELISA method and measurement of ApoE4

The sandwich ELISA method was constructed as follows. The 10 µg/ml antibody specifically reactive with all of ApoE2, ApoE3 and ApoE4 prepared in Example 2 was added to a 96-well plate for the ELISA test in an amount of 100 µl/well. After the reaction at 4°C overnight, the reaction product was blocked with a 1.0% BSA/PBS/NaN₃ solution to prepare a plate for the sandwich ELISA test. The combined product of the anti-ApoE4 fragment peptide antibody and HRP prepared in Example 1 (7) was used as a labeled antibody. The reactivity with ApoE4 was measured as follows. 100 µl of the standard solution prepared in Example 1(3) was added to the plate for the sandwich ELISA test and the mixture was reacted for one hour at 37°C. After the reaction, the plate was washed four times with 0.05% Tween 20-PBS. 100 µl of the labeled antibody was added and the mixture was reacted at 37°C for 30 minutes. After the reaction, the plate was washed six times with 0.05% Tween 20-PBS. 100 µl of a TMB solution was added and the mixture was allowed to stand in shaded conditions at room temperature for 30 minutes. The reaction was terminated with the addition of 1 N sulfuric acid to measure absorbance at 450 nm. As a result, ApoE4 could be measured specifically and a calibration curve was prepared.

### Example 4

### Enzyme immunoassay test

### (1) Sample

35 whole blood samples collected from volunteers were used for the test. A 51-fold dilution sample was prepared by adding 500 µl of a phosphate buffer solution containing 1% BSA and 0.05% Tween 20 to 10 µl of the whole blood.

### (2) Immunoreaction

100 µl of the sample prepared in (1) above was added to the plate for the sandwich ELISA test which had been prepared in Example 3. A cover was placed over the plate and the mixture was reacted for one hour at 37°C.

After one hour, the reaction mixture was washed seven times with a phosphate buffer solution containing 0.05% Tween 20. A recommended washing procedure consists of vigorously pouring the washing fluid from a wash bottle to each well of the plate to washout the wells, filling the well with the washing fluid, allowing the plate to stand for 15-30 minutes, and shaking the plate upside down to completely remove the washing solution. This washing procedure was repeated a prescribed number of times, following which the plate was dabbed onto a paper towel to completely remove the liquid from the wells. Since washing using a plate washer may not be sufficient depending on the equipment used, washing according to the above washing procedure should be repeated about three times after washing by a plate washer.

Subsequently, 100 µl of the antibody that specifically recognizes ApoE4 labeled with HRP obtained in Example 1 was added. The cover was placed over the plate and the mixture was reacted for 30 minutes at 37°C. After 30 minutes, the plate was washed nine times according to the above-mentioned procedure.

A TMB substrate solution prepared by dissolving two tablets of 1 mg TMB (3,3',5,5'-tetramethylbenzidine tablet, manufactured by Sigma-Aldrich Co.) in a buffer solution for substrate, which was prepared by mixing 5 ml of purified water and 5 ml of a buffer solution containing 0.01% hydrogen peroxide, was added in an amount of 100 µl per well. The mixture was reacted under shaded conditions at room temperature for 30 minutes. The reaction solution gradually turned blue after the addition of the TMB substrate solution. 100 µl/well of a termination solution (1 N sulfuric acid solution) was added and the side of the plate was dabbed to homogenize the mixture. After the addition of the termination solution, the reaction solution turned from blue to yellow.

The same procedure as above was carried out on one plate for samples using a standard solution 1 which can identify an ApoE4 homogeneous genotype and an ApoE4 heterogeneous genotype (1.2 µg/ml ApoE4 (a product manufactured by Wako Pure Chemical Industries, Ltd.) diluted to 51-fold with purified water), a standard solution 2 which can identify an ApoE4 heterogeneous genotype and genotypes other than ApoE4 (0.24 µg/ml ApoE4 (a product manufactured by Wako Pure Chemical Industries, Ltd.) diluted to 51-fold with purified water), and a reagent blank instead of the sample. The same procedure as in the plate for samples was carried out in a plate for sample blank.

### (3) Absorbance measurement

After the antibody reaction of (2), stains and drops water on the bottom of the plate were wiped off. After confirming absence of bubbles on the surface, the absorbances at 450 nm of the sample, standard solution 1, and standard solution 2 were measured using a reagent blank as a control within 30 minutes. The same measurement was carried out for the plate for sample blank.

The measured absorbances are defined as follows. Plate for sample
Absorbance of the sample = A
Absorbance of the standard solution 1 = B
Absorbance of the standard solution 2 = C
Plate for sample blank
Absorbance of the sample blank = D
Absorbance of the standard solution 1 = E
Absorbance of the standard solution 2 = F

### (4) Method for correcting absorbances

The value obtained by subtracting the absorbance of the plate for sample blank from absorbance of the plate for sample is regarded as the absorbance after correction.
(A) Absorbance of the sample = A - D = S
(B) Absorbance of the standard solution 1 = B - E = C1
(C) Absorbance of the standard solution 2 = C - F = C2

### (5) Method for absorbance judgment

The genotype was judged from the corrected absorbances in the above-mentioned manner based on the standard shown Table 2.

**Table 2**

| Measurement results | Judgment |
|---|---|
| S > C1 | Although the sample has an extremely high probability of having an ApoE4/E4 homogeneous genotype, a genetic test is required for the final decision. |
| C1 > S > C2 | Although the sample has an extremely high probability of having an ApoE4 heterogeneous genotype (ApoE4/E3 or ApoE4/E2 genotype), a genetic test is required for the final decision. |
| C2 > S | The sample has a genotype other than the ApoE4 genotype (ApoE3/E3, ApoE3/E2, or ApoE2/E2 genotype). |

### (6) Results

The relationship between the absorbances and judgment results and of the genotype according to the PCR method is shown in Table 2 and the genotype judgment results from the absorbances and the genotype judgment results according to the PCR method are shown in Table 3.

The judgment of genotype according to the PCR method was carried out using the method of Hixson et al. (Hixson J. E. and Vernier D. T., J. Lipid. Res. 1990, Mar, 31(3): 545-8).

**Table 3**

| Sample No. | Absorbance (x51) | Judgment according to the present invention | | Judgment according to the PCR inspection |
|---|---|---|---|---|
| S1 | 3.377 | S1>C1 | 4/4 | 4/4 |
| S2 | 3.333 | S2>C1 | 4/4 | 4/4 |
| S3 | 2.756 | S3>C1 | 4/4 | 4/4 |
| S4 | 1.214 | C1>S4>C2 | 4/2 or 4/3 | 4/2 |
| S5 | 1.531 | C1>S5>C2 | 4/2 or 4/3 | 4/3 |
| S6 | 1.218 | C1>S6>C2 | 4/2 or 4/3 | 4/3 |
| S7 | 1.033 | C1>S7>C2 | 4/2 or 4/3 | 4/3 |
| S8 | 1.726 | C1>S8>C2 | 4/2 or 4/3 | 4/3 |
| S9 | 0.312 | C2>S9 | 3/3 or 3/2 | 3/3 |
| S10 | 0.316 | C2>S10 | 3/3 or 3/2 | 3/3 |
| S11 | 0.282 | C2>S11 | 3/3 or 3/2 | 3/3 |
| S12 | 0.462 | C2>S12 | 3/3 or 3/2 | 3/2 |
| S13 | 0.152 | C2>S13 | 3/3 or 3/2 | 3/2 |
| S14 | 0.232 | C2>S14 | 3/3 or 3/2 | 3/2 |
| S15 | 1.948 | C1>S15>C2 | 4/2 or 4/3 | 4/3 |
| S16 | 0.918 | C1>S16>C2 | 4/2 or 4/3 | 4/3 |
| S17 | 0.095 | C2>S17 | 3/3 or 3/2 | 3/3 |
| S18 | 0.121 | C2>S18 | 3/3 or 3/2 | 3/3 |
| S19 | 0.085 | C2>S19 | 3/3 or 3/2 | 3/3 |
| 520 | 0.109 | C2>S20 | 3/3 or 3/2 | 3/3 |
| S21 | 0.052 | C2>S21 | 3/3 or 3/2 | 3/3 |
| S22 | 0.077 | C2>S22 | 3/3 or 3/2 | 2/3 |
| S23 | 0.151 | C2>S23 | 3/3 or 3/2 | 3/3 |
| S24 | 0.102 | C2>S24 | 3/3 or 3/2 | 3/3 |
| S25 | 0.082 | C2>S25 | 3/3 or 3/2 | 2/3 |
| S26 | 0.043 | C2>S26 | 3/3 or 3/2 | 3/3 |
| S27 | 0.070 | C2>S27 | 3/3 or 3/2 | 3/3 |
| S28 | 0.133 | C2>S28 | 3/3 or 3/2 | 3/3 |
| 529 | 0.141 | C2>S29 | 3/3 or 3/2 | 3/3 |
| S30 | 0.105 | C2>S30 | 3/3 or 3/2 | 3/3 |
| S31 | 0.100 | C2>S31 | 3/3 or 3/2 | 2/3 |
| S32 | 0.083 | C2>S32 | 3/3 or 3/2 | 3/3 |
| S33 | 0.103 | C2>S33 | 3/3 or 3/2 | 3/3 |
| S34 | 0.096 | C2>S34 | 3/3 or 3/2 | 3/3 |
| S35 | 0.111 | C2>S35 | 3/3 or 3/2 | 3/3 |

| | | | | |
|---|---|---|---|---|
| Absorbance of C1 = 2.500, Absorbance of C2 = 0.600 | | | | |

The identity rate (n=35) of the detection method of the present invention and the PCR method is shown in the following Table 4.

**Table 4**

| Genotype of ApoE | Judgment according to the present invention | Judgment according to the PCR inspection | Identity rate |
|---|---|---|---|
| ApoE4/E4 | 3 | 3 | 100% |
| ApoE4/E3 or ApoE4/E2 | 7 | 7 | 100% |
| ApoE3/E3, ApoE3/E2 or ApoE2/E2 | 25 | 25 | 100% |

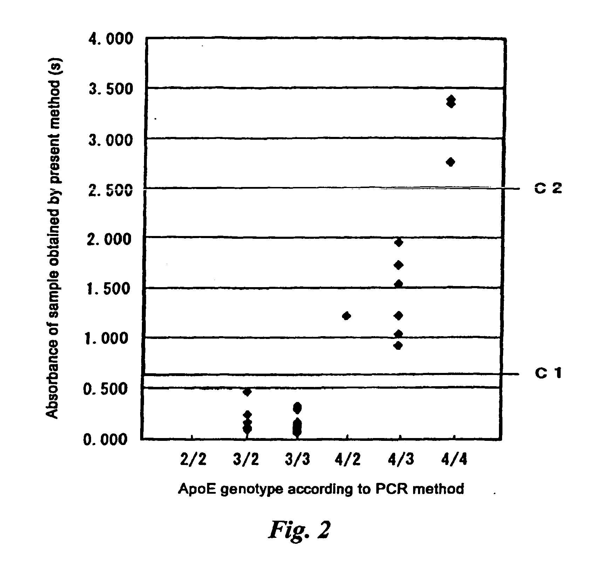

As is clear from Figure 2 and Tables 4-5, the identification result of the genotype according to the method of the present invention was exactly the same the results of identification by the PCR method.

### INDUSTRIAL APPLICABILITY

A risk factor of Alzheimer' s disease can be detected very inexpensively using the method of the present invention as compared with the conventional PCR method, since automatic colorimeters commonly used in inspecting rooms in hospitals can be applied as is without requiring any special apparatus.

In addition, since the method of the present invention can inspect genes without requiring cells possessing a gene (leukocyte etc.) that are indispensable in conventional genetic screening, but using whole blood or blood serum and plasma, the present invention can expand applicability of blood serum, plasma, and the like.

Therefore, the present invention is very effective as a means for screening a large number of samples prior to the ApoE genetic inspection which is deemed to be a definite inspection at the present time.

## Claims

1. A kit for detecting a risk factor of Alzheimer's disease containing the following reagents (a) and (b):
(a) a reagent containing an antibody specific to apolipoprotein E4, whereby said antibody specific to apolipoprotein E4 can recognize apolipoprotein E4 but does not recognize apolipoprotein E2 and apolipoprotein E3,
(b) a reagent containing an antibody specific to all of apolipoprotein E2, apolipoprotein E3, and apolipoprotein E4, which recognizes a polypeptide having a peptide sequence EKVQAAVGTSAAPVPSDNH (Sequence ID No. 3).

2. The kit according to claim 1, wherein said antibody of reagent (a) is a labeled antibody and said antibody of reagent (b) is an immobilized antibody.

3. The kit according to any one of claims 1 or 2, wherein the antibody specific to an apolipoprotein E4 is an antibody which recognizes a polypeptide having a peptide sequence ADMEDVRGRLV (Sequence ID No. 1).

4. The kit according to any one of claims 1 to 3, further comprising the following reagents (e) and (f):
(e) a standard solution 1 which can identify an ApoE4 homogeneous genotype and an ApoE4 heterogeneous genotype and
(f) a standard solution 2 which can identify an ApoE4 heterogeneous genotype and genotypes other than ApoE4.

5. A method for detecting a risk factor of Alzheimer's disease in a sample, **characterized by**:
measuring the, absorbance of a reaction product of the sample and an antibody specific to all of apolipoprotein E2, apolipoprotein E3 and
apolipoprotein E4, which recognizes a polypeptide having a peptide sequence EKVQAAVGTSAAPVPSDNH (Sequence ID No. 3);
reacting the resulting complex with an antibody specific to apolipoprotein E4, whereby said antibody specific to apolipoprotein E4 can recognize apolipoprotein E4 but does not recognize apolipoprotein E2 and apolipoprotein E3; and
detecting the genotype of apolipoprotein E4 in the sample.

6. The method according to claim 5, wherein the sample is whole blood, blood serum, or plasma.

7. The method according to claims 5 or 6, wherein said antibody specific to all of apolipoprotein E2, apolipoprotein E3 and apolipoprotein E4 is immobilized and the antibody specific to apolipoprotein E4 is labeled.

8. The method according to any one of claims 5 to 7, **characterized by** using the kit for detecting a risk factor of Alzheimer's disease according to any one of claims 1 to 4.

9. The method according to any one of claims 5 to 8, wherein the antibody specific to an apolipoproteinE4 is an antibody which recognizes a polypeptide having a peptide sequence ADMEDVRGRLV (Sequence ID No. 1).

10. The method according to any one of claims 5 to 9, wherein said step of detecting the genotype of apolipoprotein E4 in the sample is done with the guidance of an absorbance of a standard solution 1 and a standard solution 2, wherein the standard solution 1 can be used to identify an ApoE4 homogeneous genotype and an ApoE4 heterogeneous genotype, and the standard solution 2 can be used to identify an ApoE4 heterogeneous genotypes and genotypes other than ApoE4.

11. The method according to claim 10, wherein the sample is whole blood and the standard solution 1 and standard solution 2 are prepared from solutions with an ApoE4concentration respectively of 1.2 µg/ml and 0.24 µg/ml by diluting these solutions to the same dilution magnification as applied to the whole blood when measurement is performed.

12. A method for identifying a genotype of apolipoproteinE in a sample, **characterized by**
reacting the sample with an antibody specific to all of apolipoprotein E2, apolipoprotein E3, and apolipoprotein E4, which recognizes a polypeptide having a peptide sequence EKVQAAVGTSAAPVPSDNH (Sequence ID No. 3) to obtain a complex;
measuring the absorbance of a reaction product of the resulting complex and an antibody specific to apolipoprotein E4, whereby said antibody specific to apolipoprotein E4 can recognize apolipoprotein E4 but does not recognize apolipoprotein E2 and apolipoprotein E3; and
detecting the genotype of apolipoprotein E4 in the sample.

13. The method according to claim 12, wherein the antibody specific to apoliproprotein E4 is an antibody which recognizes a polypeptide having a peptide sequence ADMEDVRGRLV (Sequence ID No. 1).

14. The method according to claim 12 or 13, wherein the sample is whole blood, blood serum, or plasma.

15. The method according to any one of claims 12 to 14, wherein said antibody specific to all of apolipoprotein E2, apolipoprotein E3, and apolipoprotein E4 is immobilized and the antibody specific to apolipoprotein E4 is labeled.

16. The method according to any one of claims 12 to 15, wherein said step of detecting the genotype of apolipoprotein E4 in the sample is done with the guidance of an absorbance of a standard solution 1 and a standard solution 2, wherein the standard solution 1 can be used to identify an ApoE4 homogeneous genotype and an ApoE4 heterogeneous genotype, and the standard solution 2 can be used to identify an ApoE4 heterogeneous genotypes and genotypes other than ApoE4.

17. The method according to claim 16, wherein the sample is whole blood and standard solution 1 and standard solution 2 are prepared from solutions with an apolipoprotein E4 concentration respectively of 1.2 µg/ml and 0.24 µg/ml by diluting these solutions to the same dilution magnification as applied to the whole blood when measurement is performed.

18. The method according to any one of claims 12 to 17, **characterized by** using the kit for detecting a risk factor of Alzheimer's disease according to any one of claims 1 to 4.

## Patentansprüche

1. Kit zum Bestimmen eines Risikofaktors der Alzheimer-Krankheit, enthaltend die folgenden Reagentien (a) und (b):
(a) ein Reagens, das einen gegen Apolipoprotein E4 spezifischen Antikörper enthält, wobei der gegen Apolipoprotein E4 spezifische Antikörper Apolipoprotein E4 erkennen kann, aber Apolipoprotein E2 und Apolipoprotein E3 nicht erkennt,
(b) ein Reagens, das einen gegenüber Apolipoprotein E2, Apolipoprotein E3 und Apolipoprotein E4 spezifischen Antikörper enthält, der ein Polypeptid erkennt, das eine Peptidsequenz EKVQAAVGTSAAPVPSDNH (Sequenz ID No. 3) aufweist.

2. Kit nach Anspruch 1, worin der Antikörper des Reagens (a) ein markierter Antikörper und der Antikörper des Reagens (b) ein immobilisierter Antikörper ist.

3. Kit nach einem der Ansprüche 1 oder 2, worin der gegen Apolipoprotein E4 spezifische Antikörper ein Antikörper ist, der ein Polypeptid erkennt, das eine Peptidsequenz ADMEDVRGRLV (Sequenz ID No. 1) aufweist.

4. Kit nach einem der Ansprüche 1 bis 3, der ferner die folgenden Reagentien (e) und (f) aufweist:
(e) eine Standardlösung 1, die einen ApoE4-homogenen Genotyp und einen ApoE4-heterogenen Genotyp identifizieren kann und
(f) eine Standardlösung 2, die einen ApoE4-heterogenen Genotyp und von ApoE4 verschiedene Genotypen identifizieren kann.

5. Verfahren zum Bestimmen eines Riskikofaktors der Alzheimer-Krankheit in einer Probe, **gekennzeichnet durch**:
Messen des spektralen Absorptionsmaßes eines Reaktionsproduktes der Probe und eines gegen Apolipoprotein E2, Apolipoprotein E3 und Apolipoprotein E4 spezifischen Antikörpers, der ein Polypeptid erkennt, das eine Peptidsequenz EKVQAAVGTSAAPVPSDNH (Sequenz ID No. 3) aufweist;
reagieren lassen des resultierenden Komplexes mit einem gegen Apolipoprotein E4 spezifischen Antikörper, wobei der gegen Apolipoprotein E4 spezifische Antikörper Apolipoprotein E4 erkennen kann, aber nicht Apolipoprotein E2 und Apolipoprotein E3 erkennt; und
Bestimmen des Genotyps von Apolipoprotein E4 in der Probe.

6. Verfahren nach Anspruch 5, worin die Probe Vollblut, Blutserum oder Plasma ist.

7. Verfahren nach Anspruch 5 oder 6, worin der gegen Apolipoprotein E2, Apolipoprotein E3 und Apolipoprotein E4 spezifische Antikörper immobilisiert ist, und der gegen Apolipoprotein E4 spezifische Antikörper markiert ist.

8. Verfahren nach einem der Ansprüche 5 bis 7, **gekennzeichnet durch** die Verwendung des Kits zum Bestimmen eines Risikofaktors der Alzheimer-Krankheit nach einem der Ansprüche 1 bis 4.

9. Verfahren nach einem der Ansprüche 5 bis 8, worin der gegen Apolipoprotein E4 spezifische Antikörper ein Antikörper ist, der ein Polypeptid erkennt, das eine Peptidsequenz ADMEDVRGRLV (Sequenz ID No. 1) aufweist.

10. Verfahren nach einem der Ansprüche 5 bis 9, worin die Stufe des Bestimmens des Genotyps von Apolipoprotein E4 in der Probe durchgeführt wird auf der Basis eines spektralen Absorptionsmaßes einer Standardlösung 1 und einer Standardlösung 2, worin die Standardlösung 1 verwendet werden kann, um einen ApoE4-homogenen Genotyp und einen ApoE4-heterogenen Genotyp zu identifizieren, und die Standardlösung 2 verwendet werden kann, um einen ApoE4-heterogenen Genotyp und von ApoE4 verschiedene Genotypen zu identifizieren.

11. Verfahren nach Anspruch 10, worin die Probe Vollblut ist und die Standardlösung 1 und die Standardlösung 2 aus Lösungen mit einer ApoE4-Konzentration von 1,2 µg/ml bzw. 0,24 µg/ml durch Verdünnen dieser Lösungen auf die gleiche Verdünnung, wie sie für das Vollblut bei der Durchführung der Messung verwendet wird, hergestellt werden.

12. Verfahren zur Identifizierung eines Genotyps von Apolipoprotein E in einer Probe, **dadurch gekennzeichnet, dass**
man die Probe mit einem auf Apolipoprotein E2, Apolipoprotein E3 und Apolipoprotein E4 spezifischen Antikörper umsetzt, der ein Polypeptid erkennt, das eine Peptidsequenz EKVQAAVGTSAAPVPSDNH (Sequenz ID No. 3) aufweist, um einen Komplex zu erhalten;
Messen des spektralen Absorptionsmaßes des Reaktionsprodukts des resultierenden Komplexes und des gegen Apolipoprotein E4 spezifischen Antikörpers, wobei der gegen Apolipoprotein E4 spezifische Antikörper Apolipoprotein E4 erkennen kann, aber Apolipoprotein E2 und Apolipoprotein E3 nicht erkennt; und
Bestimmen des Genotyps von Apolipoprotein E4 in der Probe.

13. Verfahren nach Anspruch 12, worin der gegen Apolipoprotein E4 spezifische Antikörper ein Antikörper ist, der ein Polypeptid, das die Peptidsequenz ADMEDVRGRLV (Sequenz ID No. 1) aufweist, erkennt.

14. Verfahren nach Anspruch 12 oder 13, worin die Probe Vollblut, Blutserum oder Plasma ist.

15. Verfahren nach einem der Ansprüche 12 bis 14, worin der gegenüber Apolipoprotein E2, Apolipoprotein E3 und Apolipoprotein E4 spezifische Antikörper immobilisiert ist, und der gegen Apolipoprotein E4 spezifische Antikörper markiert ist.

16. Verfahren nach einem der Ansprüche 12 bis 15, worin die Stufe des Bestimmens des Genotyps von Apolipoprotein E4 in der Probe durchgeführt wird auf der Basis eines spektralen Absorptionsmaßes einer Standardlösung 1 und einer Standardlösung 2, worin die Standardlösung 1 verwendet werden kann, um einen ApoE4-homogenen Genotyp und einen ApoE4-heterogenen Genotyp zu identifizieren, und die Standardlösung 2 verwendet werden kann, um einen ApoE4-heterogenen Genotyp und von ApoE4 verschiedene Genotypen zu identifizieren.

17. Verfahren nach Anspruch 16, worin die Probe Vollblut ist und die Standardlösung 1 und die Standardlösung 2 aus Lösungen mit einer Apolipoprotein E4-Konzentration von 1,2 µg/ml bzw. 0,24 µg/ml durch Verdünnen dieser Lösungen auf die gleiche Verdünnung wie sie für das Vollblut bei der Durchführung der Messung verwendet wird, hergestellt werden.

18. Verfahren nach einem der Ansprüche 12 bis 17, **gekennzeichnet durch** Verwenden des Kits zum Bestimmen eines Risikofaktors der Alzheimer-Krankheit nach einem der Ansprüche 1 bis 4.

## Revendications

1. Kit pour la détection d'un facteur de risque de la maladie d'Alzheimer, contenant les réactifs suivants (a) et (b) :
(a) un réactif contenant un anticorps spécifique de l'apolipoprotéine E4, ledit anticorps spécifique de l'apolipoprotéine E4 pouvant reconnaître l'apolipoprotéine E4 mais ne reconnaissant pas l'apolipoprotéine E2 et l'apolipoprotéine E3,
(b) un réactif contenant un anticorps spécifique de toutes les apolipoprotéine E2, apolipoprotéine E3 et apolipoprotéine E4, qui reconnaît un polypeptide ayant une séquence peptidique EKVQAAVGTSAAPVPSDNH (Séquence ID N° 3).

2. Kit selon la revendication 1, dans lequel ledit anticorps du réactif (a) est un anticorps marqué et ledit anticorps du réactif (b) est un anticorps immobilisé.

3. Kit selon l'une quelconque des revendications 1 et 2, dans lequel l'anticorps spécifique de l'apolipoprotéine E4 est un anticorps qui reconnaît un polypeptide ayant une séquence polypeptidique ADMEDVRGRLV (Séquence ID N° 1).

4. Kit selon l'une quelconque des revendications 1 à 3, comprenant en outre les réactifs (e) et (f) suivants:
(e) une solution standard 1 qui peut identifier un génotype homogène d'ApoE4 et un génotype hétérogène d'ApoE4, et
(f) une solution standard 2 qui peut identifier un génotype hétérogène d'ApoE4 et des génotypes autres que ceux d'ApoE4.

5. Procédé pour la détection d'un facteur de risque de la maladie d'Alzheimer dans un échantillon, **caractérisé par** :
la mesure de l'absorbance d'un produit de réaction de l'échantillon et d'un anticorps spécifique de toutes les apolipoprotéine E2, apolipoprotéine E3 et apolipoprotéine E4, qui reconnaît un polypeptide ayant une séquence peptidique EKVQAAVGTSAAPVPSDNH (Séquence ID N° 3) ;
la réaction du complexe résultant avec un anticorps spécifique de l'apolipoprotéine E4, ledit anticorps spécifique de l'apolipoprotéine E4 pouvant reconnaître l'apolipoprotéine E4 mais ne reconnaissant pas l'apolipoprotéine E2 et l'apolipoprotéine E3 ; et
la détection du génotype de l'apolipoprotéine E4 dans l'échantillon.

6. Procédé selon la revendication 5, dans lequel l'échantillon est du sang entier, du sérum sanguin ou du plasma.

7. Procédé selon la revendication 5 ou 6, dans lequel ledit anticorps spécifique de toutes les apolipoprotéine E2, apolipoprotéine E3 et apolipoprotéine E4 est immobilisé et l'anticorps spécifique de l'apolipoprotéine E4 est marqué.

8. Procédé selon l'une quelconque des revendications 5 à 7, **caractérisé par** l'utilisation du kit pour la détection d'un facteur de risque de la maladie d'Alzheimer selon l'une quelconque des revendications 1 à 4.

9. Procédé selon l'une quelconque des revendications 5 à 8, dans lequel l'anticorps spécifique de l'apolipoprotéine E4 est un anticorps qui reconnaît un polypeptide ayant une séquence peptidique ADMEDVRGRLV (Séquence ID N° 1).

10. Procédé selon l'une quelconque des revendications 5 à 9, dans lequel ladite étape de détection du génotype de l'apolipoprotéine E4 dans l'échantillon est effectuée avec l'aide d'une absorbance d'une solution standard 1 et d'une solution standard 2, dans lequel la solution standard 1 peut être utilisée pour identifier un génotype homogène d'ApoE4 et un génotype hétérogène d'ApoE4 et la solution standard 2 peut être utilisée pour identifier un génotype hétérogène d'ApoE4 et des génotypes autres que ceux d'ApoE4.

11. Procédé selon la revendication 10, dans lequel l'échantillon est du sang entier et la solution standard 1 et la solution standard 2 sont préparées à partir de solutions avec une concentration d'ApoE4 respectivement de 1,2 µg/ml et 0,24 µg/ml par dilution de ces solutions au même degré de dilution que celui appliqué au sang entier lorsqu'une mesure est effectuée.

12. Procédé pour l'identification d'un génotype d'une apolipoprotéine E dans un échantillon, **caractérisé par**
la réaction de l'échantillon avec un anticorps spécifique de toutes les apolipoprotéine E2, apolipoprotéine E3 et apolipoprotéine E4, qui reconnaît un polypeptide ayant une séquence peptidique EKVQAAVGTSAAPVPSDNH (Séquence ID N° 3) pour obtenir un complexe ;
la mesure de l'absorbance d'un produit de réaction du complexe résultant et d'un anticorps spécifique de l'apolipoprotéine E4, ledit anticorps spécifique de l'apolipoprotéine E4 pouvant reconnaître l'apolipoprotéine E4 mais ne reconnaissant pas l'apolipoprotéine E2 et l'apolipoprotéine E3 ; et
la détection du génotype de l'apolipoprotéine E4 dans l'échantillon.

13. Procédé selon la revendication 12, dans lequel l'anticorps spécifique de l'apolipoprotéine E4 est un anticorps qui reconnaît un polypeptide ayant une séquence polypeptidique ADMEDVRGRLV (Séquence ID N° 1).

14. Procédé selon la revendication 12 ou 13, dans lequel l'échantillon est du sang entier, du sérum sanguin ou du plasma.

15. Procédé selon l'une quelconque des revendications 12 à 14, dans lequel ledit anticorps spécifique de toutes les apolipoprotéine E2, apolipoprotéine E3 et apolipoprotéine E4 est immobilisé et l'anticorps spécifique de l'apolipoprotéine E4 est marqué.

16. Procédé selon l'une quelconque des revendications 12 à 15, dans lequel ladite étape de détection du génotype de l'apolipoprotéine E4 dans l'échantillon est effectuée avec l'aide d'une absorbance d'une solution standard 1 et d'une solution standard 2, dans lequel la solution standard 1 peut être utilisée pour identifier un génotype homogène d'ApoE4 et un génotype hétérogène d'ApoE4, et la solution standard 2 peut être utilisée pour identifier un génotype hétérogène d'ApoE4 et des génotypes autres que ceux d'ApoE4.

17. Procédé selon la revendication 16, dans lequel l'échantillon est du sang entier et la solution standard 1 et la solution standard 2 sont préparées à partir de solutions avec une concentration d'apolipoprotéine E4 respectivement de 1,2 µg/ml et 0,24 µg/ml par dilution de ces solutions au même degré de dilution que celui appliqué au sang entier lorsqu'une mesure est effectuée

18. Procédé selon l'une quelconque des revendications 12 à 17, **caractérisé par** l'utilisation du kit pour la détection d'un facteur de risque de la maladie d'Alzheimer selon l'une quelconque des revendications 1 à 4.


### SEQUENCE LISTING

<110> Immuno-Biological Laboratories Co., Ltd.
<120> A detection reagent for Alzheimer risk factor, a detection kit for Alzheimer risk factor and a method of detecting Alzheimer risk factor with the use of them
<130> PF-020017-WO
<140>
   <141>
<150> JP2001-308465
   <151> 2001-10-04
<160> 4
(170) Patent In Ver. 2.1
<210> 1
   <211> 11
   <212> PRT
   <213> Homo sapiens
<220>
   <223> Inventor: OTA, Shigeo; MAEDA, Masahiro
<400> 1
   Ala Asp Met Glu Asp Val Arg Gly Arg Leu Val
   1 5 10
<210> 2
   <211> 12
   <212> PRT
   <213> Artificial Sequence
<220>
   <223> Description of Artificial Sequence:PEPTIDE
<400> 2
   Cys Ala Asp Met Glu Asp Val Arg Gly Arg Leu Val
   1 5 10
<210> 3
   <211> 19
   <212> PRT
   <213> Homo sapiens
<400> 3
   Glu Lys Val Gln Ala Ala Val Gly Thr Ser Ala Ala Pro Val Pro Ser
   1 5 10 15
   Asp Asn His
<210> 4
   <211> 20
   <212> PRT
   <213> Artificial Sequence
<220>
   <223> Description of Artificial Sequence:PEPTIDE
<400> 4
   Cys Glu Lys Val Gln Ala Ala Val Gly Thr Ser Ala Ala Pro Val Pro
   1 5 10 15
   Ser Asp Asn His
   20